(19) Europäisches Patentamt
European Patent Office
Office européen des brevets

(11) Numéro de publication : **0 445 025 A2**

(12) **DEMANDE DE BREVET EUROPEEN**

(21) Numéro de dépôt : **91400525.1**

(51) Int. Cl.[5] : **C07C 59/305,** A61K 31/19

(22) Date de dépôt : **27.02.91**

(30) Priorité : **27.02.90 FR 9002392**

(43) Date de publication de la demande : **04.09.91 Bulletin 91/36**

(84) Etats contractants désignés : **AT BE CH DE DK ES FR GB GR IT LI LU NL SE**

(71) Demandeur : **ADIR ET COMPAGNIE**
**1 rue Carle Hébert**
**F-92415 Courbevoie Cédex (FR)**

(72) Inventeur : **Czernecki, Stanislas**
**5 rue Bertagnes**
**F-77950 Rubelles - Maincy (FR)**
Inventeur : **Fugier, Claude**
**34 rue André Caplet**
**F-76600 Le Havre (FR)**
Inventeur : **Tsouderos, Yannis**
**80 résidence Elyséell**
**F-78170 La Celle St Cloud (FR)**

(54) **Nouveaux sels de métaux alcalinoterreux d'oxa-polyacides leur procédé de préparation et les compositions pharmaceutiques qui les contiennent.**

(57) - Composés de formule générale :

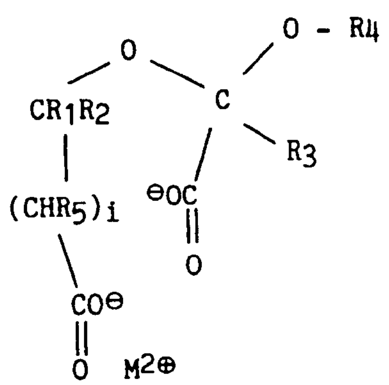

dans laquelle :
M est un métal alcalinoterreux
$R_1$, $R_2$, $R_3$, $R_4$, $R_5$, et i étant définis dans la description
- Médicaments

**EP 0 445 025 A2**

Jouve, 18, rue Saint-Denis, 75001 PARIS

# NOUVEAUX SELS DE METAUX ALCALINOTERREUX D'OXA-POLYACIDES LEUR PROCEDE DE PREPARATION ET LES COMPOSITIONS PHARMACEUTIQUES QUI LES CONTIENNENT

La présente invention concerne de nouveaux sels de métaux alcalinoterreux d'oxa-polyacides, leur procédé de préparation et les compositions pharmaceutiques qui les contiennent.

L'apport de cations divalents est indispensable à la vie des organismes animaux, notamment des mammifères donc de l'homme. Cet apport est en général assuré par l'alimentation.

Cependant, lorsque les métabolismes de l'organisme sont déséquilibrés, il est indispensable d'apporter une supplémentation en cations divalents de manière à traiter des maladies telles que : carences en oligo éléments, déficiences magnésiennes et ostéoporose.

Certaines publications de la littérature comme par exemple :

– Gastineau, Poc. Straff. Meeting Mayo Clinic **35** 105-111 (1960)

– Skoryna, Can. Med. Assoc. **125** (7), 702-712 (1981)

ou enfin,

– Skoryna, Trace Subst. Environ. Health **18**, 3-23 (1984)

font état de l'activité des lactate, gluconate et carbonate de strontium dans le traitement de l'ostéoporose.

La demanderesse a maintenant découvert de nouveaux sels de cations divalents d'oxa-polyacides dont la biodisponibilité est nettement améliorée par rapport à tous les autres sels connus.

Certains d'entre eux et en particulier les sels de strontium, de calcium et magnésium possèdent une activité surprenante sur le métabolisme osseux à savoir qu'ils activent fortement l'ostéoformation tout en inhibant parallèlement l'ostéoresorption.

Certains sels alcalins d'oxa-polyacides ont déjà été décrits dans la littérature (DE 22 48 708 B2) pour leurs propriétés détergentes sans qu'aucune propriété biologique n'ait été démontrée.

Plus spécifiquement, l'invention concerne les sels de métaux alcalinoterreux de formule générale **(I)** :

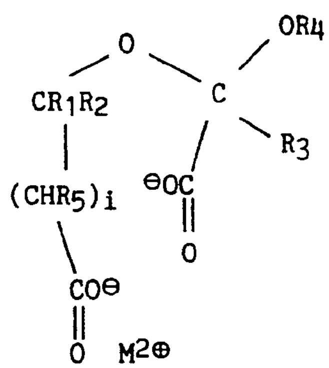

(I)

dans laquelle :

– $R_1$, $R_2$, $R_3$, $R_5$ représentent chacun indépendamment l'un de l'autre un atome d'hydrogène, un groupement alkyl inférieur de 1 à 4 atomes de carbone ramifié ou non, hydroxy, hydroxyalkyl, alkoxy, alkoxyalkyl, carboxyalkyl,

– i peut prendre les valeurs 0 et 1

– $R_4$ est un atome d'hydrogène, un groupement alkyle inférieur de 1 à 4 carbones ramifié ou non,aryle ou arylalkyle éventuellement substitué, carboxyalkyle, avec la réserve que lorsque i = 0, $R_1 = R_3 = H$, $R_2 = CH_2OH$ et lorsque i = 0, $R_1 = R_2 = R_3 = H$ alors $R_4$ est différent de $CH_3$, étant entendu que le terme substitué associé aux expressions aryle ou arylalkyle signifie que les noyaux aromatiques peuvent être substitués par un ou des groupements alkyle, nitro, alkoxy, hydroxy, halogène ou trifluorométhyle,

– M représente un métal alcalinoterreux,

– leurs isomères, épimères, diastéroisomères et énantiomères isolés ou sous forme de mélange.

Parmi les dérivés de l'invention on préfère actuellement les sels de strontium de formule générale **(I)**.

L'invention s'étend également au procédé d'obtention des composés de formule générale **(I)**,caractérisé en ce que l'on fait réagir un dérivé de formule **(II)** :

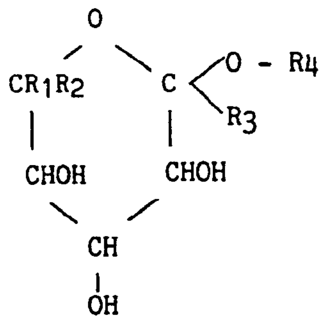

(II)

dans laquelle $R_1$, $R_2$, $R_3$ et $R_4$ ont la même signification que dans la formule **(I)**, avec de l'acide périodique de manière à obtenir un dérivé de formule générale **(III)** :

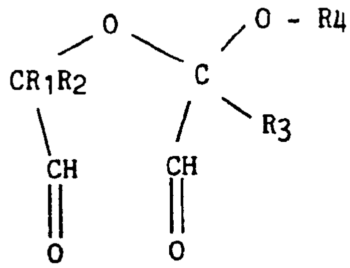

(III)

dans laquelle $R_1$, $R_2$, $R_3$ et $R_4$ ont la même signification que dans la formule **(I)**, que l'on soumet en solution d'alcool XOH à réaction d'oxydation avec le brome de manière à obtenir le polyester de formule générale **(IV)** :

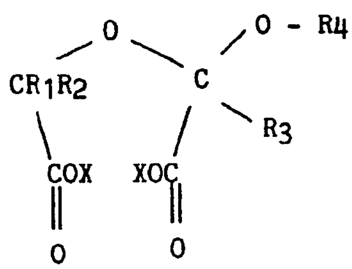

(IV)

dans laquelle $R_1$, $R_2$, $R_3$ et $R_4$ ont la même signification que dans la formule **(I)** et X est un radical alkyl inférieur de 1 à 4 atomes de carbone ramifié ou non, que l'on soumet à réaction de saponification en milieu hydroalcoolique avec un hydroxyde de métal alcalinoterreux de manière à obtenir le sel de métal alcalinoterreux correspondant de formule générale **(IA)** :

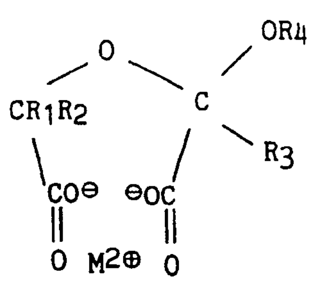

(IA)

dans laquelle $R_1$, $R_2$, $R_3$, $R_4$ et M ont la même signification que dans la formule **(I)**.

Les acides correspondants peuvent être obtenus par acidification avec de l'acide chlorhydrique.

Des cas particuliers de la présente invention sont les dérivés de formule **(I)** pour lesquels i = 1

– Ces dérivés sont préparés à partir des alkyl 4,6-0-benzylidène glucopyrannoside de formule générale **(V)** :

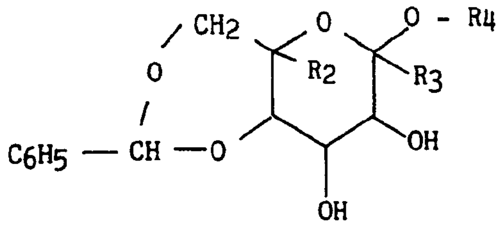

(V)

dans laquelle $R_2$, $R_3$ et $R_4$ ont la même signification que dans la formule **(I)** que l'on fait réagir avec de l'acide périodique pour obtenir le dialdéhyde que l'on déprotège ensuite pour obtenir le composé de formule générale **(VI)** :

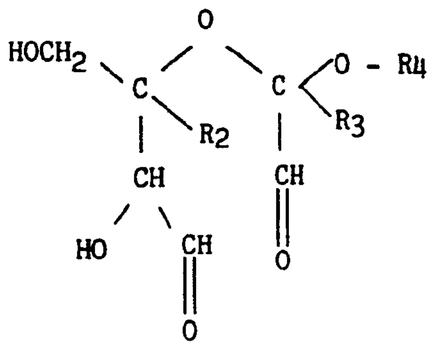

(VI)

dans laquelle $R_2$, $R_3$ et $R_4$ ont la même signification que dans la formule **(I)**, que l'on soumet en solution d'alcool XOH à réaction d'oxydation avec le brome de manière à obtenir le polyester de formule générale **(VII)**

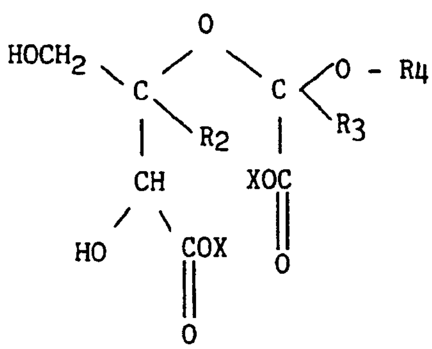

(VII)

dans laquelle $R_2$,$R_3$ et $R_4$ ont la même signification que dans la formule **(I)** et X est un radical alkyl inférieur de 1 à 4 atomes de carbone ramifié ou non que l'on soumet à réaction de saponification en milieu hydroalccolique avec un hydroxyde de métal alcalinoterreux de manière à obtenir le sel de métal alcalinoterreux correspondant de formule générale **(IB)**

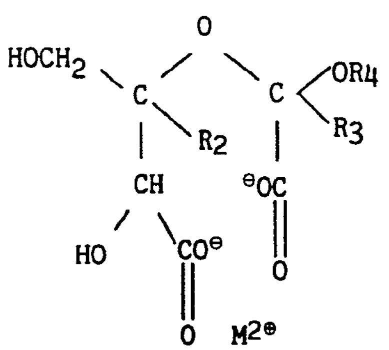

(IB)

dans laquelle $R_2$, $R_3$, $R_4$, M ont la même signification que dans la formule **(I)**

Les sels de métaux alcalinoterreux de formule générale (I) possèdent d'une part une excellente biodisponibilité et d'autre part une activité remarquable, stimulant fortement l'ostéoformation tout en inhibant parallèlement l'ostéorésorption. Ces propriétés particulièrement intéressantes permettent leur utilisation dans le traitement de certaines maladies osseuses en particulier celles résultant de troubles de la minéralisation comme l'ostéoporose ou de tumeurs à métastases osseuses. Ils peuvent également être utilisés dans le traitement du vieillissement cutané et vasculaire, des affections hépatiques et des affections dentaires. Les sels de calcium et de magnésium peuvent être utilisés dans le traitement de certaines affections, comme les carences, qui nécessitent un apport thérapeutique en cations divalents.

La présente invention a également pour objet les compositions pharmaceutiques contenant comme principe actif un des sels de formule générale (I) mélangé ou associé à un excipient inerte, non toxique et pharmaceutiquement approprié comme par exemple l'eau distillée, le glucose, le lactose, l'amidon, le talc, l'éthyl cellulose, le stéarate de magnésium, la silice colloïdale ou le beurre de cacao.

Les compositions pharmaceutiques ainsi obtenues se présentent généralement sous forme dosée et peuvent contenir selon les affectations traitées l'âge et le sexe du malade de 1 à 1000 mg de principe actif.

Elles peuvent revêtir la forme de comprimés, dragées, gélules, solutions injectables ou buvables ou de suppositoires et être, selon le cas, administrées par voie orale, rectale ou parentérale à la dose de 1 à 1000 mg de une à plusieurs fois par jour.

Les exemples suivants illustrent l'invention et ne la limitent en aucune façon.

**EXEMPLE 1 : 2-éthoxy 4-hydroxyméthyl 3-oxa pentanedioate de strontium** (composé (I) avec i = 0 , $R_1$ = $CH_2OH$, $R_2 = R_3 = H$, $R_4 = C_2H_5$, $M^{2\oplus} = Sr^{2\oplus}$obtenu à partir d'éthyl β-D-glucopyrannoside)

**Stade I :** Ethyl 2,3,4,6-tétra-0-acétyl β-D-glucopyrannoside.

Dans un réacteur sous atmosphère d'azote charger 1 litre de Toluène et 23,8 g (0,517 moles) d'éthanol anhydre. Ajouter ensuite à température ambiante la solution préalablement préparée de 200 g (0,513 moles) de β-D-glucose pentaacétate dans 2 litres de toluène. Chauffer à 40°C puis ajouter en à peu près 10 minutes, la solution préalablement préparée de 30cm³ (0,258 moles) de tétrachlorure d'étain dans 1 litre de toluène. Maintenir encore 55 minutes à 40°C, hydrolyser avec de l'eau, sécher sur sulfate de magnésium puis concentrer sous pression réduite.

Le produit brut obtenu est repris sous vive agitation par 300 cm³ d'un mélange éthanol-eau (50 - 50). La suspension est refroidie à -10°c puis filtrée sous argon.

Le solide humide est dissout à chaud (≃ 60°c) dans 800 cm³ d'un mélange éthanol-eau (60 - 40) Refroidir à -10°c, laisser cristalliser pendant 1 heure puis filtrer sous argon. Le produit est séché sous vide en présence de $P_2O_5$ jusqu'à poids constant.

On obtient ainsi 79 g (41 %) d'Ethyl 2,3,4,6-tétra-0-acétyl β-D-glucopyrannoside sous forme d'une fine poudre blanche.

Point de fusion :106°C

**Stade II** : Ethyl β-D-glucopyrannoside

Dans un réacteur sous atmosphère d'azote charger 67,68 g d'Ethyl 2,3,4,6-tétra-0-acétyl β-D-glucopyrannoside et 382 cm³ de méthanol anhydre. Agiter la suspension à température ambiante puis couler en 5 minutes 18 cm³ (0,018 moles) d'une solution molaire de méthylate de sodium dans le méthanol.

Agiter 110 minutes à 23 - 24°c puis neutraliser par addition de 21,5 g de résine amberlite $H^{\oplus}$ Eliminer les résines par filtration puis mettre à sec le milieu réactionnel sous pression réduite.

On obtient ainsi 36,5 g (100%) d'Ethyl β-D-glucopyrannoside sous forme d'une masse déliquescente jaune.

**Stade III** : 2-éthoxy 4-hydroxyméthyl 3-oxa glutaraldéhyde.

Dans un réacteur sous atmosphère d'azote charger 35 g d'Ethyl β-D-glucopyrannoside et 424 cm³ d'eau. Agiter fortement et couler rapidement à température ambiante la solution préalablement préparée de 81,2 g (0,356 moles) d'acide périodique dans 900cm³ d'eau. Agiter 3 heures à température ambiante puis refroidir et neutraliser par addition de 64cm³ de soude 10 N. Eliminer l'eau par distillation sous pression réduite.

Le solide blanc obtenu est repris dans 828cm³ d'éthanol et agité fortement pendant une heure.L'insoluble est éliminé par filtration et le filtrat éthanolique concentré à 40°c sous pression réduite.

On obtient ainsi 30,1 g (90,4 %) de 2-éthoxy 4-hydroxyméthyl 3-oxa glutaraldéhyde

**Stade IV** : 2-éthoxy 4-hydroxyméthyl 3-oxa pentanedioate de méthyle.

Dans un réacteur sous atmosphère d'azote charger 43,4 g de 2-éthoxy 4-hydroxyméthyl 3-oxa glutaraldéhyde, 220,5 g de bicarbonate de sodium et 221 $cm^3$ d'un mélange méthanol - eau (90 - 10). Agiter à température ambiante et couler en 2 heures la solution préalablement préparée de 148 g de brome dans 773 $cm^3$ d'un mélange méthanol - eau (90 - 10). Agiter ensuite 2 heures à température ambiante puis introduire en plusieurs fois 33,75 g de thiosulfate de sodium.

Filtrer la suspension obtenue, ajouter 280 $cm^3$ d'eau au filtrat et éliminer le méthanol à 40°c sous pression réduite.Extraire le concentrat par 3 fois 550 $cm^3$ de chlorure de méthylène, laver les phases d'extractions par 226 $cm^3$ d'eau puis les sécher sur sulfate de magnésium et les mettre à sec sous pression réduite.

On obtient ainsi 25,5 g (69,8 %) de 2-éthoxy 4-hydroxyméthyl 3-oxa pentanedioate de méthyle sous forme d'un produit sirupeux jaune.

RMN 200 $MH_z$ ($CDCl_3$, TMS)

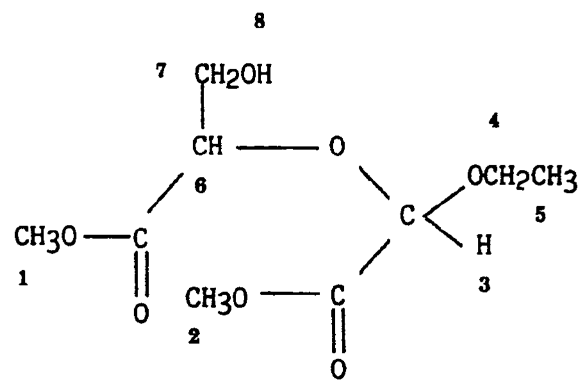

1,1 ppm triplet $CH_3$ 5

3,2 ppm multiplet CH 6

3,6 ppm multiplet $CH_2$ 4

3,6 à 3,7 ppm singulets $2CH_3$ 1 et 2

3,8 ppm multiplet $CH_2$ 7

4,3 ppm singulet CH 3

5 ppm singulet OH 8

Microanalyse :

Calculé C 45,76 H 6,82
Trouvé C 45,80 H 6,86

**Stade V** : 2-éthoxy 4-hydroxyméthyl 3-oxa pentanedioate de strontium (obtenu à partir d'éthyl β-D-glucopyrannoside).

Dans un réacteur sous atmosphère d'azote charger 10,53 g d'hydroxyde de strontium dans 120 $cm^3$ d'eau. Porter au reflux 15 minutes et éliminer l'insoluble par filtration à chaud. Additionner alors sur le filtrat la solution préalablement préparée de 9,45 g de 2-éthoxy 4-hydroxyméthyl 3-oxa pentanedioate de méthyle dans 20 $cm^3$ de méthanol. Le milieu réactionnel est porté au reflux pendant 1 heure, refroidi à 30°c, concentré sous pression réduite jusqu'aux 2/3 du volume initial puis glacé à 0°c jusqu'à fin de cristallisation du produit. Le sel de strontium est isolé par filtration puis séché sous vide à 30°c en présence de $P_2O_5$

On obtient ainsi 6,36 g (65 %) de 2-éthoxy 4-hydroxyméthyl 3-oxa pentanedioate de strontium sous forme d'une poudre blanche.

Point de fusion supérieur à 260°c

RMN 200 $MH_z$ ($D_20$, TMS)

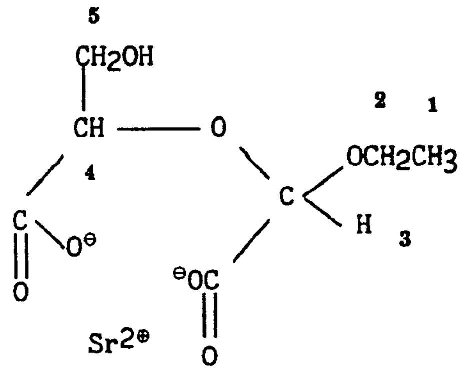

1,10 ppm triplet $CH_3$ 1

3,60 - 3,45 ppm multiplet $CH_2$ 2

3,81 ppm multiplet $CH_2$ 5

4,15 ppm multiplet CH 4

4,80 ppm singulet CH 3

Microanalyse :

Calculé C 28,42 H 4,09
Trouvé C 28,45 H 4,13
Pouvoir rotatoire :

$[\alpha]_D^{20} = + 42$ (C = 5 mg dans 1 ml d'eau)

**EXEMPLE 2 : 2-propyloxy 4-hydroxyméthyl 3-oxa pentanedioate de strontium.** (composé **(I)** avec i = 0, $R_1 = CH_2OH$, $R_2$ : $R_3$ = H, $R_4 = CH_2CH_2CH_3$, $M^{2\oplus} = Sr^{2\oplus}$ obtenu à partir de propyl β-D-glucopyrannoside).

On procède de la même manière que pour l'exemple 1 en remplacant dans le stade I l'éthanol anhydre par du n propanol anhydre.
Point de fusion supérieur à 260°c

RMN 200 $MH_z$ ($CD_3OD$, TMS)

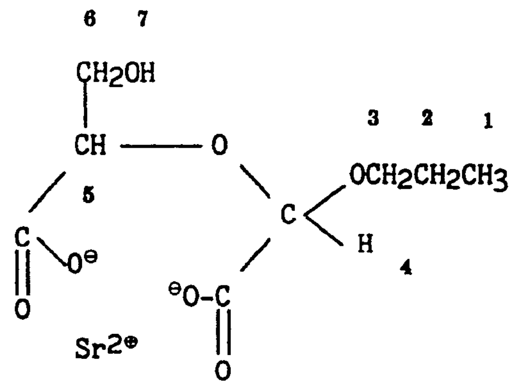

0,90 ppm multiplet $CH_3$ 1

1,6 ppm multiplet $CH_2$ 2

3,63 ppm multiplet $CH_2$ 6

3,95 ppm multiplet $CH_2$ 3

4,45 ppm multiplet CH 5

5,17 ppm singulet CH 4

5,35 ppm singulet OH 7

**EXEMPLE 3 : 2-éthoxy 4-hydroxyméthyl 3-oxa pentanedioate de strontium** (composé **(I)** avec i = 0, $R_1$ = $CH_2OH$, $R_2$ = $R_3$ = H, $R_4$ = $C_2H_5$, $M^{2\oplus}$ = $Sr^{2\oplus}$obtenu à partir d'éthyl α-D-mannopyranoside.)

**Stade I** : Ethyl α-D-mannopyranoside.

Dans un réacteur, sous atmosphère d'azote charger 100 cm³ d'éthanol absolu, 12 g de résine amberlite $H^{\oplus}$ et 18,02 g (0,100 moles) de D (+) mannose.

Le milieu réactionnel est chauffé au reflux pendant 7 heures puis refroidi et la résine éliminée par filtration. Après rinçage de la résine à l'éthanol absolu, les filtrats sont regroupés et concentrés sous pression réduite. Après mise à sec on obtient 20,9 g (100 %) d'éthyl α-D-mannopyranoside.

**Stade II** : 2-éthoxy 4-hydeoxyméthyl 3-oxa glutaraldéhyde.

Dans un réacteur sous atmosphère d'azote charger 20 g d'éthyl α-D-mannopyranoside et 242 cm³ d'eau. Agiter fortement et couler rapidement à température ambiante la solution préalablement préparée de 46,4 g (0,204 moles) d'acide périodique dans 514 cm³ d'eau. Agiter 3 heures à température ambiante puis refroidir, neutraliser par addition de 36,6 cm³ de soude 10 N et mettre à sec par distillation sous pression réduite.

Le résidu est repris dans 473 cm³ d'éthanol et agité fortement pendant une heure. L'insoluble est éliminé par filtration et le filtrat éthanolique concentré à 40° C sous pression réduite.

On obtient 16,8 g (88,3 %) de 2-éthoxy 4-hydroxyméthyl 3-oxa glutaraldéhyde.

**Stade III** : 2- éthoxy 4-hydroxyméthyl 3-oxa pentanedioate de méthyle

Dans un ballon sous atmosphère d'azote charger 0,7 g de 2-éthoxy 4-hydroxyméthyl 3-oxa glutaraldéhyde, 6 g de bicarbonate de sodium et 10 cm³ d'un mélange méthanol - eau (90 - 10). Agiter 10 minutes à température ambiante puis ajouter goutte à goutte la solution préalablement préparée de 3,2 g de brome dans 10 cm³ d'un mélange méthanol - eau (90 - 10).

Agiter ensuite 4 heures à température puis additionner 0,55 g de thiosulfate de sodium.

Filtrer la suspension, ajouter 4,5 cm³ d'eau et éliminer le méthanol à 40° C sous pression réduite. Extraire le concentrat par 3 fois 35 cm³ de chlorure de méthylène, laver les phases organiques par 10 cm³ d'eau puis

les sécher sur sulfate de magnésium et les mettre à sec sous pression réduite.

On obtient 0,75 g (100 %) de 2-éthoxy 4-hydroxyméthyl 3-oxa pentanedioate de méthyle.

Microanalyse :

Calculé C 45,76    H 6,82
Trouvé C 45,8 H    H 6,9

**Stade IV** : 2-éthoxy 4-hydroxyméthyl 3-oxa pentanedioate de strontium (obtenu à partir d'éthyl α-D-mannopyranoside)

Dans un ballon sous atmosphère d'azote charger 0,89 g d'hydroxyde de strontium dans 20 cm³ d'eau. Porter au reflux pendant 15 minutes et éliminer l'insoluble par filtration à chaud. Additionner alors sur le filtrat la solution préalablement préparée de 0,8 g de 2-éthoxy 4-hydroxyméthyl 3-oxa pentanedioate de méthyle dans 2 cm³ de méthanol. Le milieu réactionnel est porté au reflux pendant 1 heure puis mis à sec par évaporation sous pression réduite.

Le résidu de remise à sec est redissout à chaud dans 20 cm³ d'eau et la solution est filtrée à chaud avant d'être remise à sec. Le résidu obtenu est séché sous vide à 120° C pendant 5 heures.

On obtient ainsi 0,7 g (74 %) de 2 éthoxy 4-hydroxyméthyl 3-oxa pentanedioate de strontium sous forme d'une poudre blanche.

RMN 200 $MH_z$ ($D_20$, TMS)

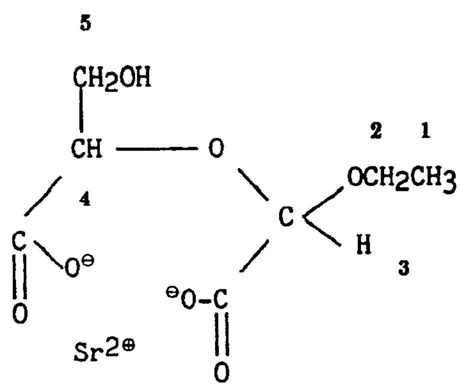

1,10 ppm triplet $CH_3$ 1
3,60 - 3,47 ppm multiplet $CH_2$ 2
3,92 - 3,77 ppm multiplet $CH_2$ 5
4,20 ppm doublet dédoublé CH 4
5,00 ppm singulet CH 3

Pouvoir rotatoire :
$[\alpha]_D^{20}$ : + 44 (C : 5 mg dans 1 ml d'eau)

**EXEMPLE 4 : 2-éthoxy 4-hydroxyméthyl 3-oxa pentanedioate de magnésium** (composé **(I)** avec i : 0, $R_1$ : $CH_2OH$, $R_2$ : $R_3$ : H, $R_4$ : $C_2H_5$, $M^{2\oplus}$ = $Mg^{2\oplus}$obtenu à partir d'Ethyl β-D-glucopyrannoside).

On procède de la même manière que pour l'exemple 1 en remplacant dans le stade V l'hydroxyde de strontium par de l'hydroxyde de magnésium.

**EXEMPLE 5 : 2-éthoxy 4-hydroxyméthyl 3-oxa pentanedioate de calcium** (composé (I) avec i = 0, $R_1$ = $CH_2OH$, $R_2 = R_3 = H$, $R_4 = C_2H_5$, $M^{2\oplus} = Ca^{2\oplus}$obtenu à partir d'Ethyl β-D-glucopyrannoside ).

On procède de la même manière que pour l'exemple 1 en remplaçant dans le stade V l'hydroxyde de strontium par de l'hydroxyde de calcium

**EXEMPLE 6 : Comprimé dosé à 500 mg de 2-éthoxy 4-hydroxyméthyl 3-oxa pentanedioate de strontium obtenu à partir d'éthyl β-D-glucopyrannoside.**

Formule pour 10000 comprimés

2-éthoxy 4-hydroxyméthyl 3-oxa pentanedioate de strontium ----- 5 Kg
Lactose ---------------------------------------------------- 2,150 Kg
Stéarate de magnésium -------------------------------------- 0,05 Kg
Silice colloïdale ------------------------------------------ 0,005 Kg
Amidon de maïs --------------------------------------------- 0,595 Kg

**EXEMPLE 7 : Gélule dosée à 5 mg de 2-éthoxy 4-hydroxyméthyl 3-oxa pentanedioate de strontium obtenu à partir d'éthyl β-D-glucopyrannoside.**

Formule pour 1000 gélules

2-éthoxy 4-hydroxyméthyl 3-oxa pentanedioate de strontium ------ 5 g
Lactose ---------------------------------------------------------- 15 g
Amidon de maïs --------------------------------------------------- 25 g
Talc ------------------------------------------------------------- 5 g

**EXEMPLE 8 : Etude pharmacologique : activité sur l'ostéoformation et l'ostéoresorption**

**a) principe**

Ostéoblaste et ostéoclaste ont entre eux des systèmes de communication encore mal connus qui expliquent le synchronisme de leurs activités permettant le renouvellement osseux.

On peut estimer que ces systèmes de communication sont conservés dans les os maintenus en culture de tissus et que l'on peut ainsi évaluer l'activité de molécules physiologiques ou thérapeutiques in vitro sur les cellules osseuses dans des conditions proches des conditions physiologiques.

L'activité sur la formation osseuse et l'activité anti-résorbante des molécules potentiellement actives comme médicaments peuvent être détectées et mesurées in vitro en culture de tissus.

**b) Méthodologie**

α) Formation osseuse

Des demi-calvarias de souriceaux nouveaux nés (1 - 2 jours) sont cultivés sur des grilles dans du milieu BGJ contenant de la proline tritiée. Les explants sont cultivés 48 h en présence du produit testé à 37°c et à la fin de la culture, les os sont lavés dans de l'acide trichloracétique contenant de la proline non radiactive pour éliminer la $^3H$-proline non incorporée.

Après lavage et dissolution dans la soude, on compte l'incorporation de marqueur dans l'os et la quantité de protéines totales. On évalue ainsi l'effet du produit sur l'incorporation de proline dans l'os en culture.

Chaque demi calvaria traitée est comparée à la demi-calvaria non traitée qui sert de controle et le rapport T/C est calculé.

$$T/C = \frac{\text{formation osseuse en présence du produit testé}}{\text{formation osseuse du contrôle}}$$

Une stimulation de la formation osseuse se traduit par une incorporation de proline tritiée supérieure chez le traité par rapport au contrôle (T/C >1).Une inhibition de la formation osseuse se manifeste par une rapport T/C <1.

Les produits ont été testés à des concentrations de $10^{-4}$, $10^{-6}$ $10^{-8}$, $10^{-10}$ et $10^{-12}$ M.

6 à 9 expériences par point ont été réalisées.

β) résorption osseuse

Des rates gestantes de 18 jours sont injectées avec 150 μcurie de $^{45}Ca$ en sous cutanée.

Au 19 ème jour les rates sont sacrifiées et les foetus sont prélevés, trempés dans un bain d'alcool à 70° puis 2 bains de PBS, tués par section de la tête. Les deux avant-bras sont disséqués, libérés de la peau, des tendons et des muscles et les extrémités cartilagineuses sont coupées. Les avant-bras sont mis en culture dans des boites de cultures 24 puits en présence de 1,5 ml de MEM durant 24 heures pour équilibration. Au bout de ces 24 heures le milieu est remplacé par 1,5 ml de BGJB supplémenté avec 10 % de sérum de veau foetal. La résorption est stimulée dans tous les échantillons par 400 mg de PTH. Les os sont cultivés durant deux périodes successives de 72 heures chacunes, les milieux de culture sont prélevés pour déterminer la quantité de $^{45}Ca$ relarguée à la fin de chacune de ces deux périodes. A la fin de la 2 ème période (7 jours après le début de la mise en culture) les os sont immergés durant 24 heures dans 1,5 ml d'acide formique 10 % et le $^{45}Ca$ relargué des os est également mesuré dans un compteur à scintillation.

Les résultats sont exprimés pour chacune des 2 périodes de culture par le rapport traité / contrôle calculé de la façon suivante :

$$\frac{\left[\dfrac{\text{radioactivité du milieu}}{\text{radioactivité relarguée de l'os}}\right]\text{traité}}{\left[\dfrac{\text{radioactivité du milieu}}{\text{radioactivité relarguée de l'os}}\right]\text{contrôle}}$$

Les produits ont été testés sur ce modèle à des concentrations de $5.10^{-3}$ et $5.10^{-4}$ M.

**c) Résulats**

α) Activité du 2-éthoxy 4-hydroxyméthyl 3-oxa pentanedioate de strontium obtenu à partir de l'éthyl β-D-glucopyrannoside sur la formation osseuse

Le 2-éthoxy 4-hydroxyméthyl 3-oxa pentanedioate de strontium exerce in vitro à la concentration de $10^{-4}$ M une stimulation importante et statistiquement significative de l'ostéoformation évaluée par incoporation de proline marquée (synthèse de collagène)

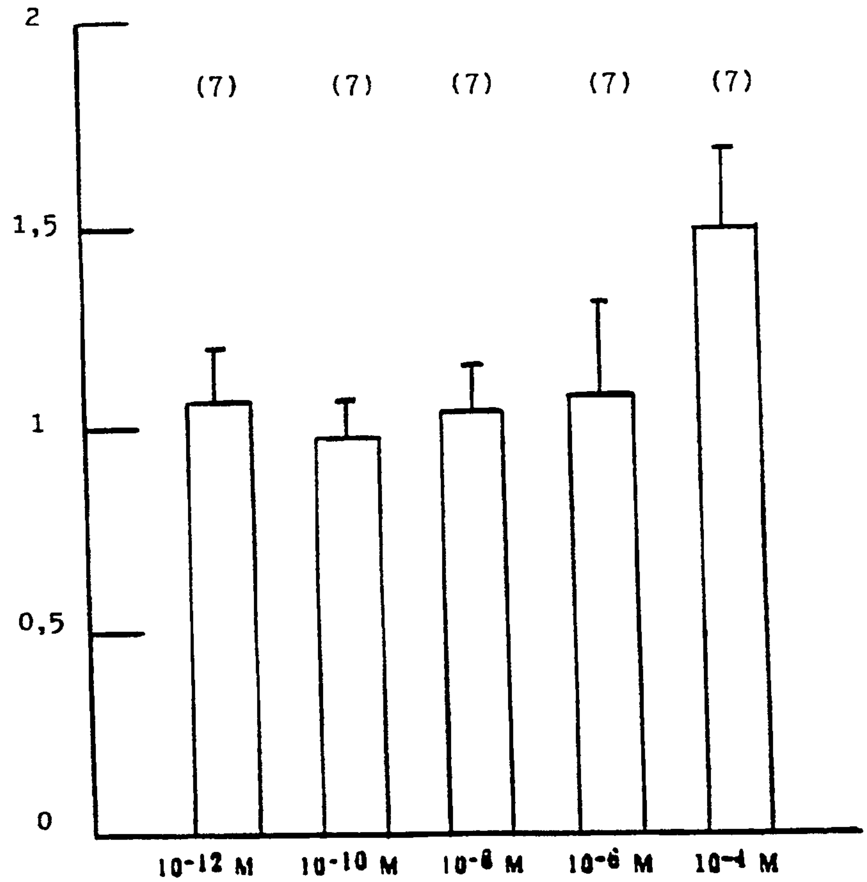


| | $10^{-12}$ M | $10^{-10}$ M | $10^{-8}$ M | $10^{-6}$ M | $10^{-4}$ M |
|---|---|---|---|---|---|
| n | 7 | 7 | 7 | 7 | 7 |
| Moyenne | 1,0582 | 0,9717 | 1,0305 | 1,0740 | 1,4961** |
| ESD | 0,1381 | 0,0926 | 0,1139 | 0,2298 | 0,1905 |
| Valeur maximale | 1,2822 | 1,0974 | 1,2092 | 1,4652 | 1,8034 |
| Valeur minimale | 0,8988 | 0,8467 | 0,8511 | 0,8433 | 1,2095 |

β) Activité du 2-éthoxy 4-hydroxyméthyl 3-oxa pentanedioate de strontium, obtenu à partir d'éthyl β-D-glucopyrannoside sur la résorption osseuse

Le 2-éthoxy 4-hydroxyméthyl 3-oxa pentanedioate de strontium **(A)** exerce une importante activité antirésorbante in vitro, statistiquement significative dès la concentration de $5.10^{-4}$ M. Cette activité est dose dépendante. Malgré la stimulation par la PTH, à la concentration de $5.10^{-3}$ M **(A)** inhibe la résorption osseuse et la ramène à un niveau nettement inférieur à la résorption spontanée.

| | Taux de résorption osseuse (%) | | T/C | |
|---|---|---|---|---|
| | J1 - J4 | J1 - J7 | J1 - J4 | J4 - J7 |
| Contrôle absolu | 6,8 ± 0,5 | 4,5 ± 1,9 | - | - |
| PTH | 14,3 ± 3,4 (a) | 37,0 ± 7,6 (b) | 1,00 | 1,00 |
| (A) 5 $10^{-4}$ | 14,5 ± 4,1 | 31,1 ± 8,34 (c) | 1,01 | 0,84 |
| (A) 5 $10^{-3}$ | 6,4 ± 0,9 (c) | 2,4 ± 0,5 (d) | 0,45 | 0,06 |

9 déterminations par point

a - différent du contrôle à $p < 0,05$

b - différent du contrôle à $p < 0,0001$

c - différent de PTH à $p < 0,05$

d - différent de PTH à $p < 0,0001$

**EXEMPLE 9 : Etude pharmacologique : Biodisponibilité**

- La détermination est faite sur des lots de 5 rats Wistar mâles
- L'administration se fait à la dose de 50 mg de Sr2⊕ par Kg sous forme de mini gelules de gélatine,
- Le dosage est effectué par spectrométrie d'absorption atomique avec ionisation en flamme pour les concentrations supérieures à 1 µg /ml et ionisation en four pour les concentrations inférieures à 1 µg /ml
- La biodisponibilité absolue du 2-éthoxy 4-hydroxyméthyl 3-oxa pentanedioate de strontium obtenu à partir d'éthyl β-D-glucopyrannoside par rapport au chlorure de strontium est de 60,5 %

**Revendications**

**1/** Sels de métaux alcalinoterreux de formule générale **(I)** :

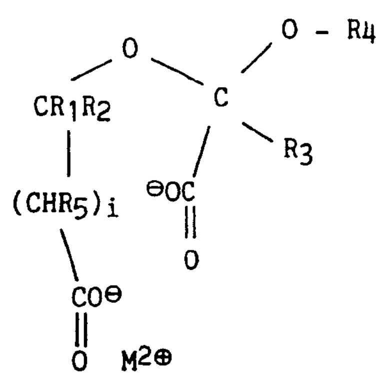

(I)

dans laquelle

- $R_1$, $R_2$, $R_3$, $R_5$ représentent chacun indépendamment l'un de l'autre un atome d'hydrogène, un groupement alkyl inférieur de 1 à 4 atomes de carbone ramifié ou non, hydroxy, hydroxyalkyl, alkoxy, alkoxyalkyl, carboxyalkyl,
- i peut prendre les valeurs 0 et 1,
- $R_4$ est un atome d'hydrogène, un groupement alkyle inférieur de 1 à 4 carbones ramifié ou non,aryle ou arylalkyle éventuellement substitué, carboxyalkyle, avec la réserve que lorsque i = 0, $R_1$ = $R_3$ = H, $R_2$ =

$CH_2OH$ et lorsque i = 0, $R_1 = R_2 = R_3 = H$ alors $R_4$ est différent de $CH_3$, étant entendu que le terme substitué associé aux expressions aryle ou arylalkyle signifie que les noyaux aromatiques peuvent être substitués par un ou des groupements alkyle, nitro, alkoxy, hydroxy, halogène ou trifluorométhyle,

– M représente un métal alcalinoterreux,

– leurs énantiomères, épimères, isomères, diastéréoisomères isolés ou sous forme de mélange.

**2/** Composés selon la revendication 1/ tel que le métal alcalinoterreux est le strontium.

**3/** Composés selon les revendications 1/ et 2/ avec i = 0, $R_1 = CH_2OH$, $R_2 = R_3 = H$, $R_4$ = alkyl inférieur de 2 à 4 atomes de carbone ramifié ou non, arylalkyl.

**4/** Composé selon les revendications 1/, 2/ et 3/ qui est le 2-éthoxy 4-hydroxyméthyl 3-oxa pentanedioate de strontium, ses isomères SS, RR, SR, RS isolés ou sous forme de mélange.

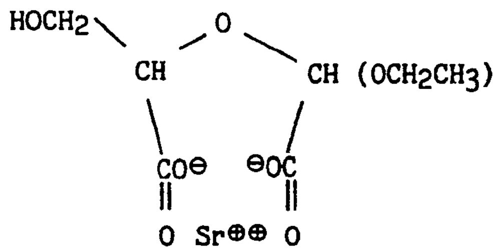

**5/** Composé selon les revendications 1/, 2/, et 3/ qui est le 2-propyloxy 4-hydroxyméthyl 3-oxa pentanedioate de strontium, ses isomères SS, RR, SR, RS isolés ou sous forme de mélange.

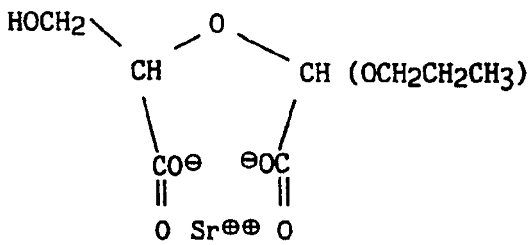

**6/** Procédé de préparation des composés de formule **(I)** caracterisé en ce que l'on fait réagir :

– soit un dérivé de formule générale **(II)**

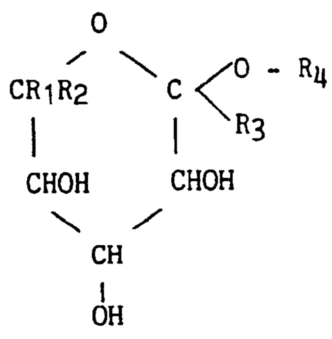

(II)

dans laquelle $R_1$, $R_2$, $R_3$, $R_4$ ont la même signification que dans la formule **(I)** avec de l'acide périodique de manière à obtenir un dérivé de formule générale **(III)**

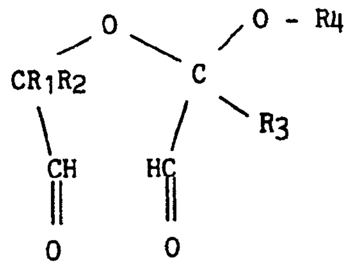

(III)

dans laquelle $R_1$, $R_2$, $R_3$, $R_4$ ont la même signification que dans la formule **(I)**, que l'on soumet en solution d'alcool XOH à réaction d'oxydation avec le brome de manière à obtenir le polyester de formule générale **(IV)**

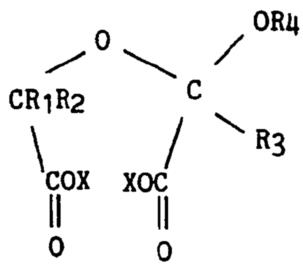

(IV)

dans laquelle $R_1$, $R_2$, $R_3$, $R_4$ ont la même signification que dans la formule **(I)** et X est un radical alkyl inférieur de 1 à 4 atomes de carbone ramifié ou non que l'on soumet à une réaction de saponification en milieu hydroalcoolique avec un hydroxyde de métal alcalinoterreux de manière à obtenir le sel de métal alcalinoterreux de formule générale **(IA)**

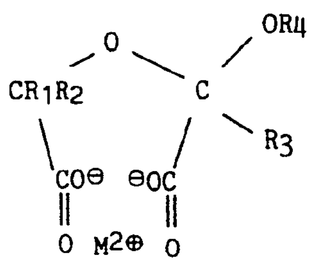

(IA)

dans laquelle $R_1$, $R_2$, $R_3$, $R_4$ et M ont la même signification que dans la fomule **(I)**
– soit un alkyl 4,6-0-benzylidène glucopyrannoside de formule générale **(V)**

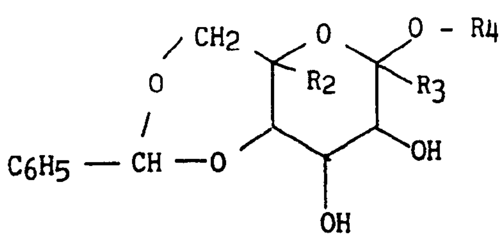

(V)

dans laquelle $R_2$, $R_3$ et $R_4$, ont la même signification que dans la formule **(I)** que l'on fait réagir avec de l'acide périodique pour obtenir après déprotection le compose de formule générale **(VI)** :

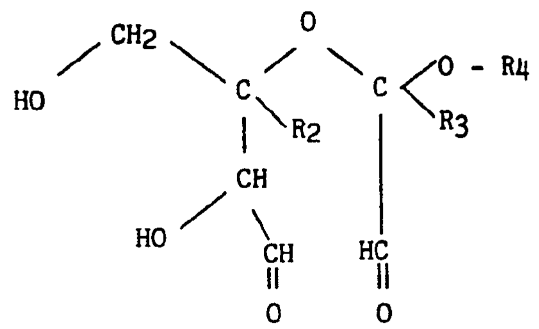

(VI)

dans laquelle $R_2$, $R_3$ et $R_4$ ont la même signification que dans la formule **(I)**, que l'on soumet en solution d'alcool XOH à réaction d'oxydation avec le brome de manière à obtenir le polyester de formule générale **(VII)**

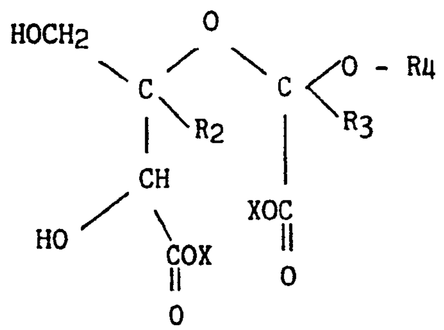

(VII)

dans laquelle $R_2$, $R_3$, $R_4$ ont le même signification que dans la formule (I) et X est un radical alkyl inférieur de 1 à 4 atomes de carbone ramifié ou non que l'on soumet à réaction de saponification en milieu hydroalcoolique avec un hydroxyde de métal alcalinoterreux de manière à obtenir le sel de métal alcalinoterreux correspondant de formule génerale **(IB)**

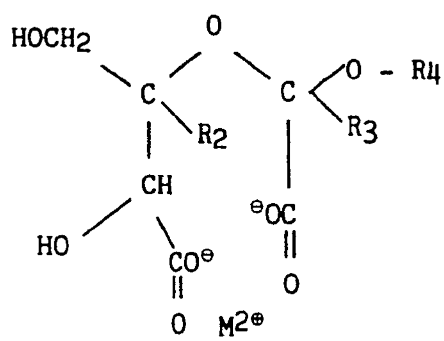

(IB)

dans laquelle $R_2$, $R_3$, $R_4$, M ont la même signification que dans la formule **(I)**.

**7/** Compositions pharmaceutiques contenant comme principe actif un sel de métal alcalinoterreux selon une quelconque des revendications 1/ à 5/ seul ou en combinaison avec un ou plusieurs excipients ou véhicules inertes, non toxiques pharmaceutiquement acceptables.

**8/** Composition selon la revendication 7/ contenant le 2-éthoxy 4-hydroxyméthyl 3-oxa pentanedioate de strontium comme principe actif.

**9/** Compositions pharmaceutiques selon les revendications 7/ et 8/ utiles pour le traitement des maladies osseuses dont l'ostéoporose, du vieillissement cutané et vasculaire, des affections dentaires et des maladies hépatiques.

**Revendications pour l'Etat contractant suivant: ES**

**1/** Procédé de préparation des sels de métaux alcalinoterreux de formule générale **(I)** :

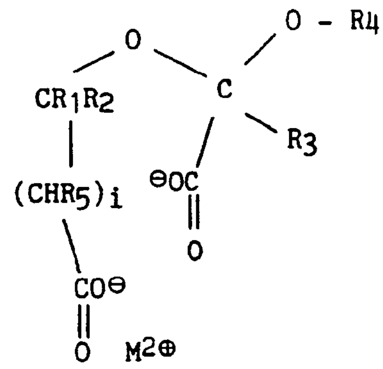

(I)

dans laquelle

- $R_1$, $R_2$, $R_3$, $R_5$ représentent chacun indépendamment l'un de l'autre un atome d'hydrogène, un groupement alkyl inférieur de 1 à 4 atomes de carbone ramifié ou non, hydroxy, hydroxyalkyl, alkoxy, alkoxyalkyl, carboxyalkyl,
- i peut prendre les valeurs 0 et 1,
- $R_4$ est un atome d'hydrogène, un groupement alkyle inférieur de 1 à 4 carbones ramifié ou non,aryle ou arylalkyle éventuellement substitué, carboxyalkyle, avec la réserve que lorsque i = 0, $R_1 = R_3 = H$, $R_2 = CH_2OH$ et lorsque i = 0, $R_1 = R_2 = R_3 = H$ alors $R_4$ est différent de $CH_3$, étant entendu que le terme substitué associé aux expressions aryle ou arylalkyle signifie que les noyaux aromatiques peuvent être substitués par un ou des groupements alkyle, nitro, alkoxy, hydroxy, halogène ou trifluorométhyle,
- M représente un métal alcalinoterreux,

ainsi que de leurs énantiomères, épimères, isomères, diastéréoisomères isolés ou sous forme de mélange, caracterisé en ce que l'on fait réagir

- soit un dérivé de formule générale **(II)**

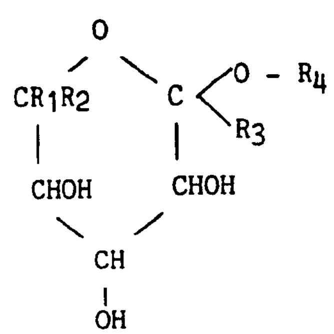

(II)

dans laquelle $R_1$, $R_2$, $R_3$, $R_4$ ont la même signification que dans la formule **(I)** avec de l'acide périodique de manière à obtenir un derivé de formule générale **(III)**

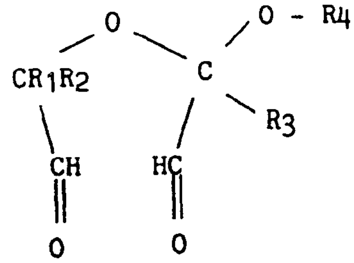

(III)

dans laquelle $R_1$, $R_2$, $R_3$, $R_4$ ont la même signification que dans la formule **(I)**, que l'on soumet en solution d'alcool XOH à réaction d'oxydation avec le brome de manière à obtenir le polyester de formule générale **(IV)**

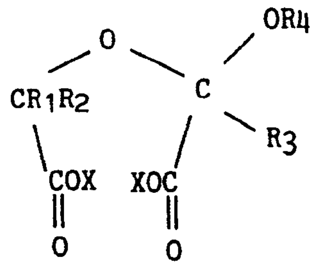

(IV)

dans laquelle $R_1$, $R_2$, $R_3$, $R_4$ ont la même signification que dans la formule **(I)** et X est un radical alkyl inférieur de 1 à 4 atomes de carbone ramifié ou non que l'on soumet à une réaction de saponification en milieu hydroalcoolique avec un hydroxyde de métal alcalinoterreux de manière à obtenir le sel de métal alcalinoterreux de formule générale **(IA)**

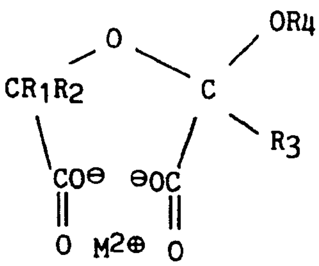

(IA)

dans laquelle $R_1$, $R_2$, $R_3$, $R_4$ et M ont la même signification que dans la formule **(I)**
– soit un alkyl 4,6-0-benzylidène glucopyrannoside de formule générale **(V)**

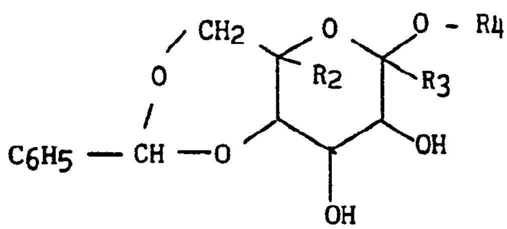

(V)

dans laquelle $R_2$, $R_3$ et $R_4$, ont la même signification que dans la formule **(I)** que l'on fait réagir avec de l'acide périodique pour obtenir après déprotection le composé de formule générale **(VI)** :

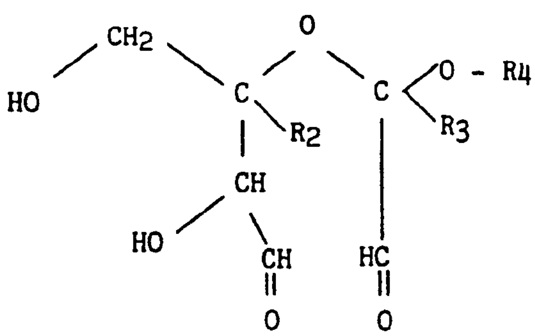

(VI)

dans laquelle $R_2$, $R_3$ et $R_4$ ont la même signification que dans la formule **(I)**, que l'on soumet en solution d'alcool XOH à réaction d'oxydation avec le brome de manière à obtenir le polyester de formule générale **(VII)**

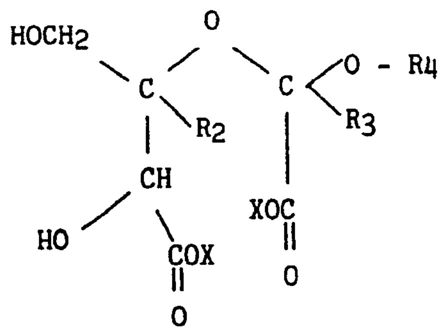

(VII)

dans laquelle $R_2$, $R_3$ , $R_4$ ont le même signification que dans la formule **(I)** et X est un radical alkyl inférieur de 1 à 4 atomes de carbone ramifié ou non que l'on soumet à réaction de saponification en milieu hydroalcoolique avec un hydroxyde de métal alcalinoterreux de manière à obtenir le sel de métal alcalinoterreux correspondant de formule générale **(IB)**

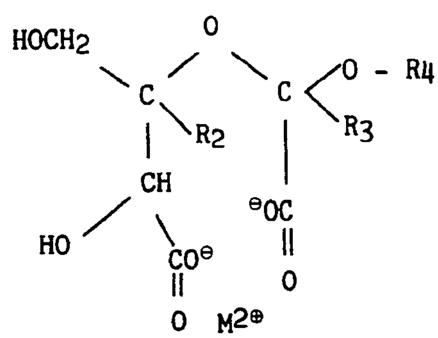

(IB)

dans laquelle $R_2$, $R_3$, $R_4$, M ont la même signification que dans la formule **(I)**.

**2/** Procédé de préparation selon la revendication 1/ du composé de formule générale (I) qui est le 2-éthoxy 4-hydroxymethyl 3-oxa pentanedioate de strontium, ses isomères SS, RR, SR, RS isolés ou sous forme de mélange.

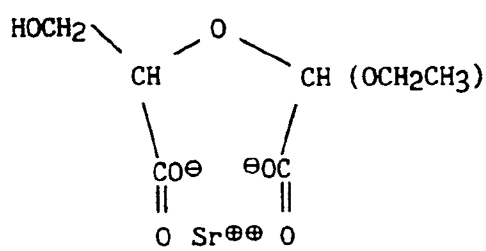

**3/** Procédé de préparation selon la revendication 1/, du composé de formule générale (I) qui est le 2-propyloxy 4-hydroxyméthyl 3-oxa pentanedioate de strontium, ses isomères SS, RR, SR, RS isolés ou sous forme de mélange.

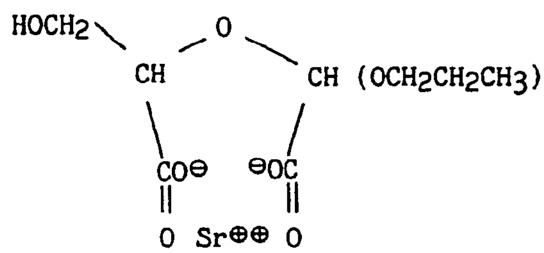

**4/** Procédé de préparation de compositions pharmaceutiques contenant comme principe actif un sel de métal alcalinoterreux obtenu selon les revendications 1/ à 3/ seul ou en combinaison avec un ou plusieurs excipients ou véhicules inertes, non toxiques pharmaceutiquement acceptables.

**5/** Procédé de préparation de composition selon la revendication 4/ contenant le 2-éthoxy 4-hydroxymethyl 3-oxa pentanedioate de strontium comme principe actif.

**6/** Procédé de préparation selon les revendications 4/ et 5/ de compositions pharmaceutiques utiles pour le traitement des maladies osseuses dont l'ostéoporose, du vieillissement cutané et vasculaire, des affections dentaires et des maladies hépatiques.

## Revendications pour l'Etat contractant suivant: GR

**1/** Procédé de préparation des sels de métaux alcalinoterreux de formule générale **(I)** :

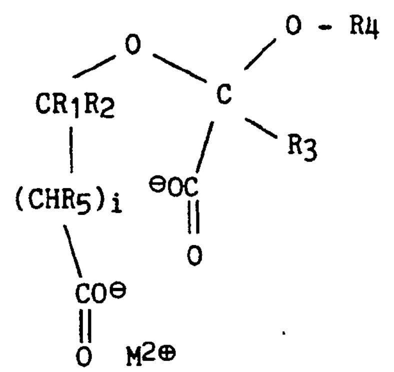

(I)

dans laquelle

– $R_1$, $R_2$, $R_3$, $R_5$ représentent chacun indépendamment l'un de l'autre un atome d'hydrogène, un groupement alkyl inferieur de 1 à 4 atomes de carbone ramifie ou non, hydroxy, hydroxyalkyl, alkoxy, alkoxyalkyl, carboxyalkyl,

– i peut prendre les valeurs 0 et 1,

– $R_4$ est un atome d'hydrogène, un groupement alkyle inférieur de 1 à 4 carbones ramifié ou non,aryle ou arylalkyle éventuellement substitué, carboxyalkyle, avec la réserve que lorsque i = 0, $R_1 = R_3 = H$, $R_2 = CH_2OH$ et lorsque i = 0, $R_1 = R_2 = R_3 = H$ alors $R_4$ est différent de $CH_3$, étant entendu que le terme substitué associé aux expressions aryle ou arylalkyle signifie que les noyaux aromatiques peuvent être substitués par un ou des groupements alkyle, nitro, alkoxy, hydroxy, halogène ou trifluorométhyle,

– M représente un métal alcalinoterreux,

ainsi que de leurs énantiomères, épimères, isomères, diastéréoisomères isolés ou sous forme de mélange, caractérisé en ce que l'on fait réagir

– soit un dérivé de formule générale **(II)**

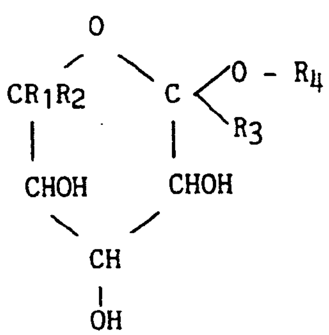

(II)

dans laquelle $R_1$, $R_2$, $R_3$, $R_4$ ont la même signification que dans la formule **(I)** avec de l'acide périodique de manière à obtenir un dérivé de formule générale **(III)**

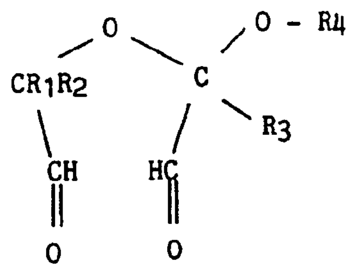

(III)

dans laquelle $R_1$, $R_2$, $R_3$, $R_4$ ont la même signification que dans la formule **(I)**, que l'on soumet en solution d'alcool XOH à réaction d'oxydation avec le brome de manière à obtenir le polyester de formule générale **(IV)**

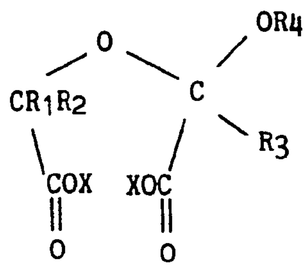

(IV)

dans laquelle $R_1$, $R_2$, $R_3$, $R_4$ ont la même signification que dans la formule **(I)** et X est un radical alkyl inférieur de 1 à 4 atomes de carbone ramifié ou non que l'on soumet à une réaction de saponification en milieu hydroalcoolique avec un hydroxyde de métal alcalinoterreux de manière à obtenir le sel de métal alcalinoterreux de formule générale **(IA)**

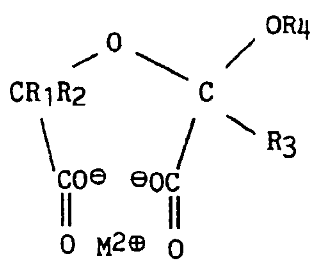

(IA)

dans laquelle $R_1$, $R_2$, $R_3$, $R_4$ et M ont la même signification que dans la formule **(I)**
– soit un alkyl 4,6-0-benzylidène glucopyrannoside de formule générale **(V)**

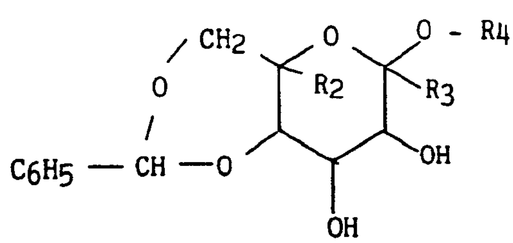

(V)

dans laquelle $R_2$, $R_3$ et $R_4$, ont la même signification que dans la formule **(I)** que l'on fait réagir avec de l'acide périodique pour obtenir après déprotection le composé de formule générale **(VI)** :

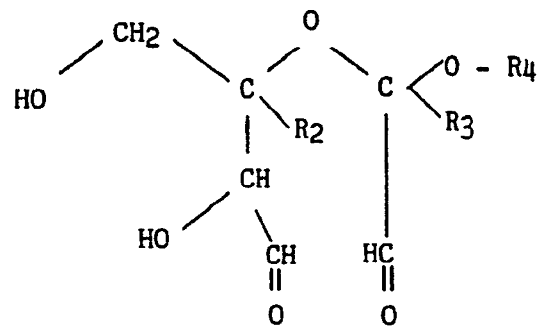

(VI)

dans laquelle $R_2$, $R_3$ et $R_4$ ont la même signification que dans la formule **(I)**, que l'on soumet en solution d'alcool XOH à réaction d'oxydation avec le brome de manière à obtenir le polyester de formule générale **(VII)**

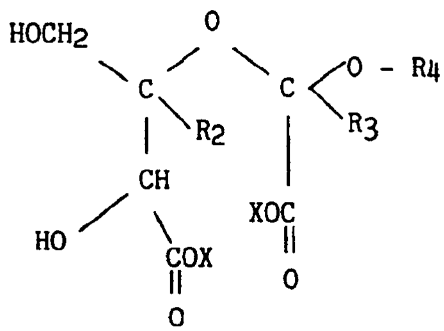

(VII)

dans laquelle $R_2$, $R_3$, $R_4$ ont le même signification que dans la formule (I) et X est un radical alkyl inférieur de 1 à 4 atomes de carbone ramifié ou non que l'on soumet à réaction de saponification en milieu hydroalcoolique avec un hydroxyde de métal alcalinoterreux de manière à obtenir le sel de métal alcalinoterreux correspondant de formule générale **(IB)**

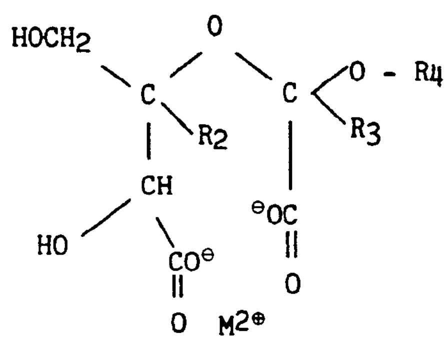

(IB)

dans laquelle $R_2$, $R_3$, $R_4$, M ont la même signification que dans la formule **(I)**.

**2/** Procédé de préparation selon la revendication 1/ du composé de formule générale (I) qui est le 2-ethoxy 4-hydroxyméthyl 3-oxa pentanedioate de strontium, ses isomères SS, RR, SR, RS isolés ou sous forme de mélange.

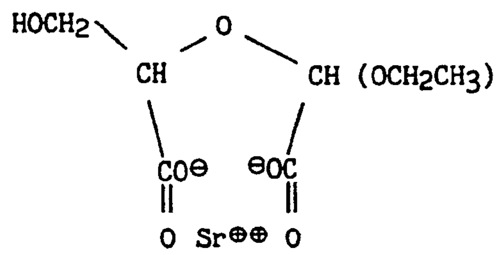

**3/** Procédé de préparation selon la revendication 1/, du composé de formule générale (I) qui est le 2-propyloxy 4-hydroxyméthyl 3-oxa pentanedioate de strontium, ses isomères SS, RR, SR, RS isolés ou sous forme de mélange.

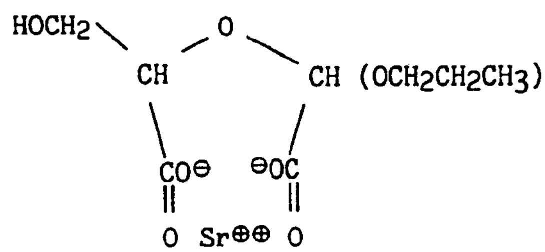

**4/** Procédé de préparation de compositions pharmaceutiques contenant comme principe actif un sel de métal alcalinoterreux obtenu selon les revendications 1/ à 3/ seul ou en combinaison avec un ou plusieurs excipients ou véhicules inertes, non toxiques pharmaceutiquement acceptables.

**5/** Procédé de préparation de composition selon la revendication 4/ contenant le 2-éthoxy 4-hydroxymethyl 3-oxa pentanedioate de strontium comme principe actif.

**6/** Procédé de préparation selon les revendications 4/ et 5/ de compositions pharmaceutiques utiles pour le traitement des maladies osseuses dont l'ostéoporose, du vieillissement cutané et vasculaire, des affections dentaires et des maladies hépatiques.